# EUROPEAN PATENT APPLICATION

(11) **EP 1 974 766 A1**
(43) Date of publication of application: **01.10.2008**
(21) Application number: 07012664.4
(22) Date of filing: 28.06.2007
(51) Int. Cl.: A61M 25/10, A61M 16/04

(54) **Multilayer cuff**

(30) Priority: 29.03.2007 EP 07006580
(71) Applicant: Hussain Khan, Zahid, Tehran (IR); Rahim Sobhani, Nader, Tehran (IR); Ali Noyan Ashraf, Mohammad, Tehran (IR)
(72) Inventor: Hussain Khan, Zahid, Tehran (IR); Rahim Sobhani, Nader, Tehran (IR); Ali Noyan Ashraf, Mohammad, Tehran (IR)
(74) Representative: Goddar, Heinz J.

(57) **Abstract**

Cuff (C₁,C₂) with a plurality of different layers (L₁,L₂,L₃,Lₘ,Lₙ), each of which being impermeable, porous or semi-permeable and/or mechanically and/or electrically and/or optically perforated, use of a cuff according to any one of the preceding claims singly or in various numbers on ETTs e.g. SLT (Single Lumen Tube), Double lumen, Bronchial Blocker, RAE Laser tubes, Tracheostomy tubes, supra glottic instruments and etc.

## Description

A cuff is a plastic balloon. Cuffs have been utilized in various medical devices, and are used for fixation and as a guide for placement of catheters. Conventional cuffs are composed of just a single layer.

### Background of the invention:

The technique entitled, "Intubation", which includes inserting a cuffed or cuffless ETT (Endotracheal tube) into the respiratory channel, i.e. the trachea of the patient, is used to promote mechanical ventilation in surgical or intensive care settings. It is needless to mention that the cuffless tubes mentioned above are used in the case of little children.

This method is also used as a means in keeping the respiratory channels open, which is of vital importance during surgeries. During the intubation, the ETT and the cuffs act as foreign objects in the respiratory passages, leading to the intolerance of the patients and consequently to reactions in the air passages.

Extubation, or in other terms removal of the ETT(s) at the end of the anesthesia of the patient, also leads to reactions that are technically called "Emergence reactions" including hypertension, tachycardia, dysrythmia, increases in IOP (Intra Ocular Pressure), ICP (Intra Cranial Pressure), bucking or straining, bronchospasm, laryngospasm, glottic spasm and so on that can lead to myocardial ischemia, defective arterial oxygenation, and vomiting, which may in turn lead to serious repercussions endangering the life of the patient. Other disadvantages resulting from the application of the presently available ETTs include tearing of stitches and bleeding as a result of bucking or straining by the patient. The intolerance of the ETTs can also lead to self-extubation of these devices by the patients that may lead to a critical situation, which if not attended promptly and urgently, can lead to the catastrophic outcomes. The main reason behind such emergence reactions is the intolerance of the ETT owing to lighter planes of anesthesia towards the end of surgery and prior to extubation. The increase in blood pressure, pulse rate, IOP, and/or ICP of the patients, suffering from underlying coronary artery disease or who have undergone eye or intracranial surgeries and pregnancy induced hypertension (PIH) may lead to disastrous outcomes (Fagan C,et al; The effects of intracuff lidocaine on endotracheal-tube-induced emergence phenomena after general anesthesia. Anesth Analg 2000;91:201-5, Diachun CD, et al. Suppression of cough during emergence from general anesthesia: laryngotracheal lidocaine through a modified endotracheal tube. J Clin Anesth 2001; 13:447-57, Gonzalez RM, et al. Prevention of endotracheal tube-induced coughing during emergence from general anesthesia. Anesth Analg 1994; 79:792-5, Huang CJ. Hsu YW. Chen CC.. Wei TT; et al, Prevention of coughing induced by endotracheal tube during emergence from general anesthesia--a comparison between three different regimens of lidocaine filled in the endotracheal tube cuff.Acta Anaesthesiologica Sinica.36(2):81-6, 1998 Jun, Marino Paul L, the ICU book, Lippincott Williams & Wilkins, Philadelphia, 2ed Edition, 1998, pp: 130-137, Miller Ronald D, Anesthesia, NY, Churchill Livingstone 2005, pp 1632, 1650). Inadvertent or unanticipated extubation may lead to laryngospasm, bronchospasm, hypoxia and/or pulmonary edema commonly encountered in all patients specially those with hyper reactive airway diseases or bronchial asthma. Also post surgical stridor, sore throat and irritable cough may also be caused due to the application of the cuffed ETTs (Fagan C et al; The effects of intracuff lidocaine on endotracheal-tube-induced emergence phenomena after general anesthesia. Anesth Analg 2000;91:201-5).

Many methods have been used to overcome or at least lessen these reactions in patients, which include systemic administration of opioids, sedatives, hypnotics (Martin D, et al; Low dose fentanyl blunts circulatory response to endotracheal intubation. Anesth Analg. 1982.61 (8): 680-684), β blockers (Mangusson J, et al; Hemodynamic effects of pretreatment with metoprolol in hypertensive patients undergoing surgery. Br J Anesth. 1986. 58(3):260), Na nitoprusside (Stoelting PK; Attenuation of blood pressure response to laryngoscopy end tracheal intubation with nitroprusside. Anesth Analg 1977.58: 116-117) local anesthetics (Yukioka H, et al; Intravenous lidocaine as a suppressant of coughing during tracheal intubation "Anesth Analg 1985.64 (12) 1184 -1192, Bidwal AV, et al Blood pressure and rate response to endotracheal extubation with and without prior injection of lidocaine: Anesth 1974. 46:315-6, Steinhaus JE, et al, A study of IV lidocaine as a suppressant of cough reflex: Anesthesiology 1963. 24:285 - 90, Gefke K, et al; Lidocaine given IV as a suppressant of cough and laryngospasm in connection with extubation after tonsillectomy" Acta Anesthesiol Scan 1983-27 (2): 111-112, Leicht P, et al Does IV lidocaine prevent laryngospasm after extubation in children? "Anesth - Analg 1985: 1193-1196, Soltani HA, et al; The effect of different lidocaine application methods on postoperative cough and sore throat. "J Clin Anesth, 2002. Feb 14 (1): 15-18), topically applied local anesthetics (Soltani HA, et al; The effect of different lidocaine application methods on postoperative cough and sore throat. "J Clin Anesth, 2002. Feb 14 (1): 15-18), intra-cuff administration of lidocaine, intra-cuff administration of warmed and alkalinized lidocaine (Gonzalez RM, et al. Prevention of endotracheal tube-induced coughing during emergence from general anesthesia. Anesth Analg 1994; 79:792-5, Altinta F, et al. Lidocaine 10% in endotracheal tube cuff and bucking: Eur J Anestheiol 2000 JUL 17(7): 436-4, 2 Estebe JP, et al. Alkainization of intracuff lidocaine improves endotracheal induced Emergence phenomena: Anesthesia- Analgesia, 2002, Jan 94(1): 227-30), and new designs of ETT(s) such as Mallinckrodt ETT (Marcus V, et al. The efficacy of topical lidocaine administration via the Mallinckrodt Hi-Lo jet tube in attenuating straining and cuffing upon emergence from general anesthesia: Anesth Analg 1994; 32:276-9) for applying local anesthetics to tracheal mucosa, using 10% lidocaine and etidocaine sprays through some channels on the ETT and the LITA ETT (Gonzalez RM, et al. Prevention of endotracheal tube-induced coughing during emergence from general anesthesia. Anesth Analg 1994; 79:792-5 Diachun CA. et al. Suppression of cough during emergence from general anesthesia: laryngotracheal lidocaine through a modified endotracheal tube. Journal of Clinical Anesthesia. 13(6):447-51, 2001 Sep.UI: 11578890). Tolerance of the ETTs in conscious patients with intact airway reflexes of the entire respiratory system (e.g. in the intensive care units), is almost impossible. Thus opioids and other anesthetic drugs are administered so as to increase the threshold of tolerance of the tubes by the patients and prevent self-extubation and/or other possible reflexes. This however leads to a prolonged stay in the intensive care unit (ICU) and thus results in an increased risk of complications such as infections, pressure sores and narrowing of the wind pipe-the trachea.

### Description of the invention:

Thus the main object of the present invention is to provide a cuff built or installable on cuffed or cuffless ETTs or any other cuffed devices, and the application thereof to overcome the general problems of the traditional cuffed ETTs mentioned above, that is the intoleration of the devices after the wearing off of anesthesia and hence leading to the above mentioned physiological reactions.

This object is achieved by a cuff with a plurality of different layers, preferably double layer, each of which being impermeable, porous or semi-permeable and/or mechanically and/or electrically and/or optically perforated.

The present invention provides a multi-layer cuff or several single layer cuffs that override one another, the physical and/or chemical properties of each layer of which is/are different and are used as a single cuff. The cuff is used to spray any drugs of desire to its neighboring tissues through holes that are designed to be present at its outer layer. The cuff is applicable on different conventional cuffed or cuffless devices, and especially on endo tracheal tubes (ETTs). The latter mentioned application reduces the intolerability of cuffs in the patients after the wearing off of the anesthesia, but the other applications also have their own desirable effects based on the device, case, and drug to be sprayed. In the case of using the multi-layer cuff of the present invention in other devices, the injection of anesthetics or other medications also leads to different advantages during the treatment.

Furthermore, the present invention provides the use thereof according to claim 12 and a method of applying said cuff according to claim 13.

According to an embodiment of the present invention, the cuff of the present invention, may also be built or installed in any numbers and/or positions on other cuffed oral or nasal ETTs and or devices like RAE, bronchial blockers, double lumen tubes, laser SLTs (Single Lumen Tube), supra glottic instruments (specially during extubation), tracheostomy tubes or in order to perform irrigation such as Broncho Alvolar Lavage (BAL), etc and instruments that may be designed in the future.

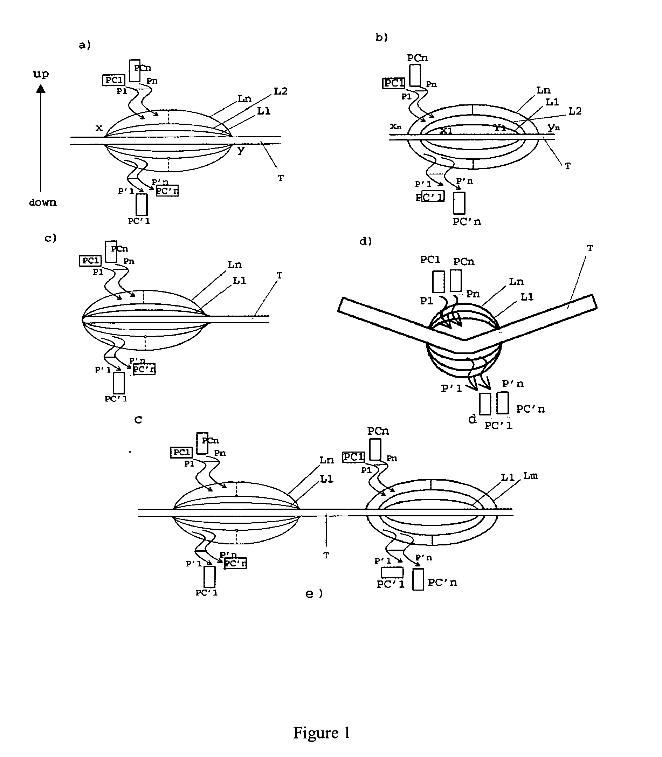

The cuff of the present invention can have different numbers of layers (Figure 1 a-e) with different physical and/or chemical properties, different shapes and or different installation or fixation properties, and different number of inlets and out lets based on the goal of its application (Figure 1, a-d).

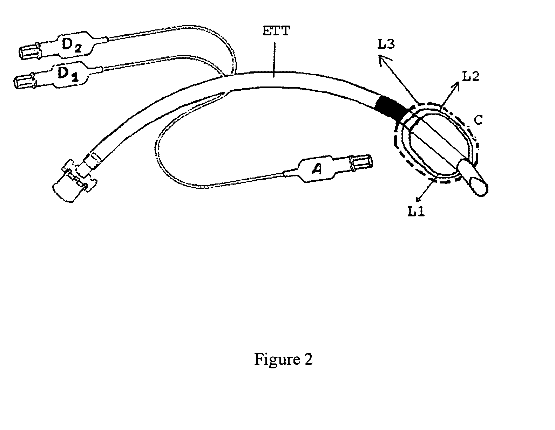

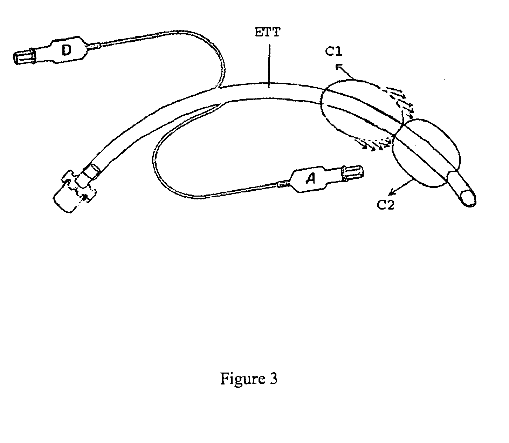

In case of the application of the cuff system of the present invention on ETT(s) (Figures 2-4), the cuff will preferably be composed of three layers (L₁, L₂, and L₃ figure 2) or three overriding single layer cuffs forming a multilayer cuff with three pilot balloons (A, D₁ and D₂, figure 2), each of which can serve different goals, but some of the major purposes of which may be as follows:
1- The innermost layer (L₃, Figure 2) or cuff is preferred to have an intact impermeable membrane and serves to fix the tube in position of desire.
2- The middle layer (L₂, Figure 2) or cuff is preferred to be semi-permeable or agent specific and can serve as a depot of drug to be slowly released outwards especially in ICU patients.
3- The outermost layer (L₁, Figure 2) or cuff has pores and/or holes in it to serve as immediate releasing depot of drug outwards to bring into direct contact with the mucosal layers of the tracheal lining.
   The preferred number of layers in the cuff of the present invention is two, and in the case of the preferred double layer cuff the outer layer is preferred to be perforated, while the inner layer is intact.
   In case other designs are used in which more than one cuff is present in the structure of the cuff-bearing device, each cuff can have a different number of layers (Figure 1 e, layers L₁-Lₙ and L₁-Lₘ, where m and n are not necessarily equal).
   In the case with two cuffs L₁ and L₂ (double layer cuff) shown in Figure 3 one (distal cuff) (C₁, Figure 3) of the cuffs is perforated for the spraying of the drug, while the other one (proximal cuff) (C₂, Figure 3) is used for the fixation purpose.

According to another embodiment of the present invention, the cuff(s) of the present invention and their pilot balloons P₁-Pₙ and P₁'-Pₙ' (Figure 1), can be designed to be built fixedly on the ETTs and/or devices mentioned above, or they can be built in a modular, i.e. as separate parts, way so they can be installed on the conventional, existing devices for the above mentioned applications, or any other possible future applications.

According to a preferred embodiment of the present invention, the cuffs are preferred to be built fixedly on the ETT(s) and or devices of desire.

According to another embodiment of the present invention, the cuff(s) can be built separately or modular, as an independent apparatus to be fixed on the devices of desire, or even in the form of single layer cuff(s) that can have the ability of being used as the layers of the multilayer cuff, finally resembling the multilayer cuff of the present invention.

According to another embodiment of the present invention, in the case of designing the modular layers (which can be single layer cuffs themselves) to be mounted on the existing devices, the cuffs may also, as in the case of the fixed multilayer cuffs built on the body of the apparatus, have different symmetric and asymmetric shapes with different pore and/or hole characteristics located symmetrically or asymmetrically at one side, both sides, and be of different sizes, shapes and diameters.

According to another embodiment of the present invention, each layer of the multilayer cuff of the present invention can also be made of different materials, and have different orifices in order to have the capability to be installed over different locations on a linear (Figure 1a) - c)), bent (Figure 1d)), and/or circular tube T with connections to other tubes.

According to a preferred embodiment of the present invention and on the contrary to the existing cuffs the cuffs and pilot balloons are preferred to be made of the same flexible material.

According to another embodiment of the present invention, the installation location of the overriding layers of the cuff(s) of the present invention on ETTs, or any of the above-mentioned devices, can be either the same as the layers below (Figure 1a) x-y), or on different symmetric and/or asymmetric positions (Figure 1b), x₁-y₁ to xₙ-yₙ).

One or several cuff(s), according to other preferred embodiments of the present invention, can be installed on any desired device in which case the layer properties of each cuff can be the same or different (Figure 1e)).

In the case of the application of several multilayer cuffs on a device it is preferred that the layer properties of the two or more cuffs C₁ and C₂ (Figure 3) be different.

According to a more preferred embodiment of the present invention, in case of preferring to install the cuff before the main cuff of the device of desire (e. g. ETT [Figures 3]), the proximal cuff (that is the one before the original or main one) (C1, Figure 3) is preferred to be perforated to let the drugs of desire leave it during the application. This will help the drug flow downward (in the case of ETTs), anesthetizing the areas in contact with the main cuff (i.e. the cuff closer to the end of the tube) (C2, Figure 3).

As mentioned above, the cuff system of the present invention can be installed proximal (C1, figure 3) to the fixation cuff (C2, figure 3), in which case the preferred method of application of the system is as follows:
After intubation and well before the patient is aware again, the distal cuff is deflated (C₂, figure 3) after suctioning the oropharyngeal area while the drug of desire (e. g. local anesthetic), which is injected to the proximal cuff (C₁, figure 3)leaves it through its holes. Following the installation of the anesthetic into the proximal cuff, and spraying most of the drug from the pores, the distal cuff (C₂, figure 3) is re-inflated via its own pilot balloon for re-fixation of tracheal tube and preparing for extubation. The proximal cuff is preferably composed of two layers (double layer) or two single layer cuffs which override one another. In both cases the internal layer is intact and is used to help to squeeze the remaining drugs to reach the tracheal mucosa and anesthetizing it, although this stage may not be necessary.

According to another embodiment of the present invention, in any case (e. g. in the case of the multilayer cuff(s) built on ETTs), each layer of the cuffs may have its own shape and/or characteristics, and also its own number of inlets and outlets or it may be designed to jointly use the inlet(s) and/or outlet(s) of the other fixed or mounted layer(s).

According to another embodiment of the present invention, the modular cuff(s) of the present invention may be used for single use or repeated uses, depending on the material, type, application, or other effective factors.

As already mentioned above, all or some desired embodiments of the present invention can be applied on ETT(s) and/or any other apparatus in which one or several cuffs are used for the fixation, and/or drug delivery.

According to other embodiments of the present invention, in all of the above mentioned applications and any other application not mentioned above, the use of the proposed cuff system, and the consequent target delivery of anesthetics and/or other drugs of desire, will lead to a higher level of toleration of the cuffed systems, or other desired therapeutic effects caused due to the application of the desired drugs.

According to another embodiment of the present invention, the cuff systems of the present invention can have other therapeutic applications rather than delivery of drugs, such as expansion of blocked and/or narrowed arteries, or extraction of objects blocking and/or fixed within different channels.

According to another embodiment of the present invention, the double or multilayer cuffs of the present invention can also be used for the delivery of drugs such as thrombolytic materials through an appropriately designed cuffed located at the distal end of the Fugarty (Fogarty) catheters, and vasoconstrictors/sclerotherapy in Blackmore catheters, or treatment of varicose veins in limbs and so on and in sampling pulmonary secretions from intubated patients suspected of having pneumonias, cystic fibrosis or the device can be used for long term instillation of drugs e.g. for intubated ICU patients.

According to another embodiment of the present invention, the multilayer-cuffed systems resulting from the embodiments of the present invention can also be used in conditions like angiofibroma, esophageal varices and hemorrhoids by anesthetizing the target area initially, and later on injecting the sclerosing agents. Similarly the application of the multilayer cuff(s) also appears to be of value in coronary artery stenosis causing their dilatation and concomitant role and feasibility of drug injection such as heparin, fibrinolytics and anti thrombotic agents, etc.

The embodiments of the present invention can also be used for the application of double cuffed tubes in various sizes & shapes for injecting drugs (e.g. vasoconstrictors) locally to patients with epistaxis anywhere in the nasopharyngeal area.

One of the most preferred applications of the cuff(s) of the present invention is their application on ETTs. The resulting ETT(s) (Figures 2-4) have multilayer cuff(s), each layer having separate inlet and outlet pilot balloons. The different layers of the cuff may also have pores and holes of different sizes and shapes or may be semi-permeable. The holes and pores of each cuff can have different sizes, and the layers may be used in a way that any different layer of any pore and/or hole size may be located below or over any other layer of interest.

The new multi-layer cuffed ETT can have any number of required pilot balloons that can be used for different purposes including the inflation of the cuffs and or injecting or circulating different substances in their liquid and/or gaseous states.

The multi-layer cuffed ETT of the present invention may have different sizes and shapes, can be made using different polymeric, organic and or composites and can be used for any other purposes in different parts of the body, in order to perform the normal functions of normal cuffed instruments, or other functions such as local delivery and spraying of single, or drug mixtures.

The multi-layer cuffed ETT can be designed to have one or more cuff(s), each having different sizes and dimensions (Figure 3). Each cuff may also be designed to have symmetrical or asymmetrical shapes and /or layer properties (Figure 1a) - 1e)), in order to meet the requirements of any special or general case.

Therefore it is an object of the present invention to design an ETT to solve the problems encountered during the application of the traditional ETTs, which were mentioned above.

According to an embodiment of the present invention, the new cuff system of the ETT tube can handle the local delivery of any anesthetic, or any other medication, in the periphery of the cuffs in the traditional ETT, multiple cuffs in any especially tailored ETT, or any other desired locations in promoting drug delivery.

According to another preferred embodiment of the present invention, the multilayer cuff system for local delivery of the desired drugs comprises one or several multilayer cuffs, each layer of which can have one or several pilot balloons (Figure 1, P₁-Pₙ and P'₁-P'ₙ) that can be used for expansion of the cuff, or its outer layer, for the injection of one or several drugs of liquid, gas, suspension, or gel drugs for different purposes, or in order to wash one or all of the cuffs for any further applications.

According to another embodiment of the present invention the different layers (L₁-Lₙ, Figure 1) of the cuff(s) may be made of different biocompatible or non-biocompatible organic, inorganic, composite, or polymeric compounds where the non-biocompatible layers can be used provided that they do not contact the internal organs of the body, or any sensitive external parts, either as the lower layers of the cuff, or with some means in avoiding direct contact.

According to another embodiment of the present invention, the material selected for the layers in the cuffs can be either porous materials of different pore sizes, the pore sizes of which may be selected so that they are either equal or different in different layers of the cuff(s), or the layers are mechanically and/or electrochemically and/or optically and/or using any other suitable techniques perforated in the same or different, homogenous or non-homogenous way in order to let the drug(s) inside each of which enter the outer cuff or finally the tissues in contact, in a reasonable distance or in the vicinity of the cuff(s).

According to another preferred embodiment of the present invention, any of the layers of cuff(s) of the present designed cuff(s), are of the so-called high-volume and low-pressure type in order to avoid probable damages to the ETT, to make it easier to leave the anesthesia state, and also to make it easier for the patient to tolerate the tube.

It is noteworthy that ETT(s) always have a fixed conformation which is in a form that always a certain half circle of all ETTs and hence a certain half circle of the cuff(s) can be in contact with the lower part of the inner layer of the respiratory channel (not shown), (Figure 1a) - e)), the lower hemisphere of the cuff) and the other half circle of the tube is, as a result, in contact with upper part of the inner layer of the respiratory channel. As a result the distribution of the pores and/or holes on the cuff(s) may be required to be designed in certain patterns depending on the type and physical state of the drugs to be used.

According to what is said about the size, and/or number of the holes and/or pores on the parts of the cuff of the present invention in touch with respiratory channel are preferred to be chosen so that in case of the application of liquid drug(s), which will gather at the lower half of the cuff (Figure 1a) - e), the lower hemisphere) due to gravity, a moderate amount of the drug can leave the lower holes.

It is also preferred that the number of holes in upper hemisphere (Figure 1a) - e), the upper hemisphere) of cuff be more or their sizes be larger, according to most preferred embodiments of the present invention, in order to increase the area to which the delivery of the drug is desired.

Taking this into account, according to another embodiment of the present invention, the material of the different layers of cuff(s), or the perforating means can be chosen so that the holes or pores in the lower half circle of the tubes are smaller than those of the upper circle. This will, in case liquid drugs are filled in one or some of the layers of the cuff(s), avoid more diffusion of the drug, which is accumulated at the lower half circle of the cuff(s) due to gravitational effects, to the lower part of the inner layer of the respiratory channel.

The application of the cuff system of the present invention is preferred to be according to one of the following conformations.

### A-Multilayer-multi-cuffed devices

In this case it is preferred that more than one of the multilayer cuff(s) of the present invention be installed or built on the device of desire (Figure le), and figure 3)

In the case of the existence of more than one cuff, at least one of the cuff(s) have one or several layers (L₁-Lₙ, Figure 1e)), and each layer can have pores/holes on it or may be semi-permeable. (L₁-Lₙ Figure 3).

According to an embodiment of the present invention, in the case of the application of more than one cuff, the number of cuff(s) is preferred to be two (Figure 3). Preferably, at least one of said two cuffs comprises at least two layers.

According to another preferred embodiment of the present invention, in the case of the application of more than one cuff, one of them can have pores and/ holes in their layer or middle one can be semi-permeable or agent specific.

According to another preferred embodiment of the present invention in the case of the application of two cuffs of a device (especially an ETT) it is preferred that one of the cuff(s) (namely the proximal cuff C₁ (Figure 3) is perforated, while the other one the distal cuff C₂ (Figure 3) is impermeable and is used for fixation process. In this case the perforated proximal cuff is the means for the delivery of the anesthetic or other drugs to the neighboring tissues of itself and also the neighboring tissues of the fixation or distal cuff.

According to another preferred embodiment of the present invention, the perforated distal cuff is preferred to be located before the fixation proximal cuff. The proximal cuff is the perforated one, while the distal one is the fixation cuff. This will cause, as will be described later, the drugs of desire get the target tissues of the fixation cuff better according to a preferred application process of the present invention.

According to the most preferred method of the application of this construction after intubation and well before the patient is aware again, the distal cuff is deflated (C₂, figure 3) after suctioning the oropharyngeal area while the drug of desire (e. g. local anesthetic), which is injected to the proximal cuff (C₁, figure 3) leaves it through its holes. Following the installation of the anesthetic into the proximal cuff, and spraying most of the drug from the pores, the distal cuff (C₂, figure 3) is re-inflated via its own pilot balloon for re-fixation of tracheal tube and preparing for extubation. The proximal cuff is preferably composed of two layers (double layer) or two single layer cuffs which override one another. In both cases the internal layer is intact and is used to help to squeeze the remaining drugs to reach the tracheal mucosa and anesthetizing it, although this stage may not be necessary.

According to a preferred method of the application of the multi (and preferably double or triple) cuff(ed) ETT (Figure 3) of the present invention, the following method can be used for the delivery of the drug to the target tissue:
One should first fill the drug delivery distal cuff C₁ (Figure 3) with a proper locally administrable anesthetic drug or a mixture thereof, at any optimum temperature and/or pressure a proper time before the extubation and before the effects of the anesthetics on the patient wears off. It is noteworthy that at this stage the fixation or proximal cuff C₂ (Figure 3), is inflated. The fixation or proximal cuff C₂ (Figure 3) is then deflated. A suction step must be performed before deflation to avoid probable risks.

The drug in the delivery distal cuff C₁ is then forced to leave the pores of the drug delivery or proximal cuff. Air injection can be applied to facilitate the exit of the drugs. The gravitational effects cause the drug to move downwards, leading to the delivery of the drug to the lower parts of the respiratory passage (not shown), including the parts in touch with the fixation cuff C₂. The next step, that maybe not necessary, includes deflation and then reinflation of the inner layer of C₁ (Figure 3), which not only helps the remaining drugs leave the outer layer of the proximal cuffs, but can also serve as the original cuff in conventional ETT(s). Finally the fixation cuff C₂ (Figure 3) is re-inflated to perform its inherent role of fixation. And of course the device is removed after the local anesthetics anesthetize the tissues, reducing the intoleration.

### B- One multilayer cuff

In this conformation it is preferred that the cuff is either built or installed on a cuff-less device in which case its structure should be designed to perform any action done by the conventional device in addition to the new applications of the present invention, or on a conventional device.

According to the mentioned embodiment of the present invention the number of the layers in the cuff, and the physical and/or chemical characteristics and properties of the cuff layers should be chosen to meet the specified goals.

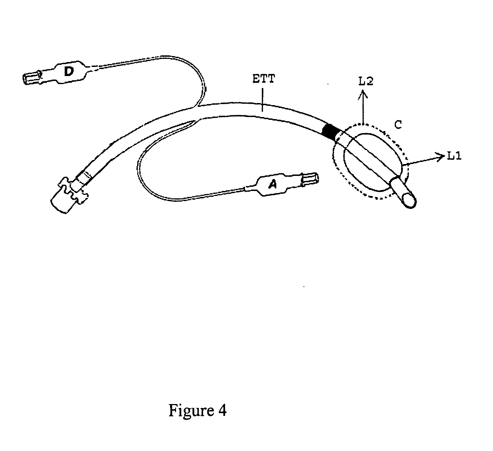

The inner layer L₁ (Figures 2 and 4) of the cuffs (no matter the second layer is built or installed on the ETT or the other devices), is used to perform the conventional role of the cuff(s) in the ETT, and also to help the residues of the drug in the outer layer of the cuff(s) be better sprayed to the target tissue.

The multilayer-cuffed ETT of the present invention can be used for elective and urgent/emergent surgeries due to its innumerable advantages elaborated above. According to another preferred method of the application of a single cuffed ETT of the present invention, the disadvantages of the conventional tubes are avoided and the best performance of the new multilayer cuffed systems are achieved by: First filling the outer layer (L₃, L₂ Figure 2) and (L₂ figure 4) of the cuff(s) with a proper locally administrable anesthetic drug or a mixture thereof through one of its pilot balloons (A, D₁ and/or D₂ Figure 2 and D, Figure 4), a proper time before the extubation and before the effects of the anesthetics on the patient wears off, and then, letting a locally administrable anesthetic drug leave it through the holes and/or pores present on it. The next step that maybe not necessary, includes deflation and then reinflation of the inner layer (L₁, Figure 2) which not only helps the remaining drugs leave the outer layer of the cuff(s), but can also serve as the original cuff in conventional ETT(s). In situations where deflation of the inner cuff is unwarranted, injection of air into the perforated cuff can help in complete emptying of the cuff containing of drug.

The drugs that can fulfill the goals of the present invention include a mixture of drugs or other gaseous or liquid materials.

In case the pores in the layers of the cuff(s) are small, or in case the diffusion of the drug, through the pore and/or holes of the layers is relatively slow, the drugs can be injected to the outer layer after prior preheating, to a required temperature.

According to another embodiment of the present invention the overall system can be automatized.

According to another embodiment of the present invention, regarding the fact that ETT(s) or other devices, on which the cuff system of the present invention is applied, the patients do not suffer the disadvantages of the conventional ones, more cuffs can be built on the tubes and devices using the present invention, to achieve any further desired goals.

Each layer of the multilayer cuff(s) of the present invention has its own pilot balloons (P₁-Pₙ and P'₁-P'ₙ Figure 1, a) - e)), connected to pressure release valves (PC₁-PCₙ and PC'₁-PC'ₙ Figure 1, a) - e)) (The presence of the mentioned pressure release valve(s) for the inlet(s) and/or outlet(s) of the cuff(s) helps avoid the development of excessive pressure in the layer(s) of the cuff which may be dangerous.

The existence of these pressure release valves provides the option of controlling the pressure of each cuff, which depending on the application may have certain tolerability limits. Each outlet and/or inlet can be controlled using different apparatus ranging from hypodermal syringes, to dosing pumps, warmers or infusers.

The goals of the present invention are further reached by preferably designing two cuffs on the body of any ETT, laser SLT (Single Lumen Tube), double lumen(s), Bronchial Blocker(s), RAE, Tracheostomy tubes, Supra glottic instruments, or any other apparatus in which cuff(s) are used either for fixation purposes and used for or needed for the delivery of drugs. The innermost layer L₁ (Figure 1a) - e)) is preferred to be gas and/or liquid impermeable, while the outermost layer Lₙ (Figure 1a) - e)) may have pores and/or holes on it to let the drug leave them and reach the tissues in touch or in proximity with the cuff.

According to another embodiment of the present invention the path from the pilot balloon to the cuff and even as farther as the tip of the tube may either be porous and/or perforated so that the injected drugs are also delivered to the tissues in farther vicinity from the fixation cuff. The ETT's lumen can be designed to have two layers (or multilayers) to facilitate the exit of desired drugs through the pores in outer layer in order to anesthetize all over the tracheal tract, and even between the cuff(s) and the bevel.

The cuffs may have different shapes, sizes and/or positions. The shape of the cuffs may range from cylindrical, to round to elliptical, while they are preferred to have the typical elliptical structure as in typical ETTs, while depending on the application of the multilayer cuffs(s) in other apparatus they may change in shape to meet the required needs.

The position of the cuff(s) may vary from the beginning Figure 1a) and b) to the end of the apparatus, (Figure 1c)), or at bent parts of the tubes (Figure 1d)), depending on the needs.

In the case of the typical ETTs, the cuffs are preferred to be positioned in the normal location of cuff(s) as in typical ETTs, while depending on the application of the multilayer cuff(s) in other apparatus they may change in position to meet the required goals.

As mentioned before the layers of each cuff may be impermeable, porous or perforated, or semi membranous or a combination thereof, depending on the material to be used in their construction, and each layer may be made of the same or different materials than the other layers.

In case the layers are porous or are mechanically, electronically, optically (or according to any other proper methods) perforated, the size and dissemination of the pores and/or holes on them can be all the same or can vary within each layer and/or from one layer to the other.

In case the sizes of the pores and/or holes in the different layers are different, any sequence of layers may be used in the design, depending on the probable application of the apparatus.

Different composite, polymeric, organic and/or inorganic material or a mixture thereof can be used in the construction of the cuff(s), and/or the whole apparatus.

The different layers of the cuff(s) may also be chosen from materials, or the pore sizes may be chosen in a range that the layers are selectively permeable to only one, or a certain group of drugs.

As regards the present invention all biocompatible and/or mechanically flexible and/or durable polymeric materials can be used in order to make the cuff(s).

It should also be noted that in case more tissues are to be subjected to drug delivery, the intratracheal path from the pilot balloon to the layers of the cuff can also be perforated in order to increase the delivery area.

It is preferred to use poly vinyl chloride membranes as the layers of the cuff(s), and in case the cuff(s) or any of their layers are required and/or desired to be drug permeable they should better be perforated using different mechanical, electrical, and/or optical methods.

The size of the holes can be in the range of 0.001 micrometer to 5 mm, preferably insulin needle bevel size and the number of pores all over the cuff can be in range of two to an infinite number in the case of the present invention. Different drugs and/or mixture thereof can be delivered to the target tissues that are touching the cuff(s) and/or in their vicinity, via being injected to a layer of the cuff, and leaving the layer through its pores and or holes.

In the case of the present invention the drugs are preferred to be one or a combination of drugs such as local anesthetics (LA) alkalinized LA and aerosolized drugs and or other tracheal mucos compatible drugs or gasses, as anesthetics so that the areas around the cuff are anesthetized and the pain is eliminated.

According to the most preferred embodiment of the present invention, local anesthetics such as lidocaine are injected to the second layer of the cuff to reduce the mentioned pain and intolerance.

The mentioned drugs can be used with or without pretreatments like heating to a desired and suitable temperature, or being mixed with one or a combination of reagents in order to increase the efficiency of their diffusion to the tissues in touch with the cuff.

The embodiments of the present invention, and the efficiency of the drug delivery is so valuable that the drugs can be used, in contradistinction to the previous efforts, in dosages far lower than the toxic dosage of the drugs, especially the local anesthetics, which eliminates the risks due to the high dosages or the probable burst of the cuffs in the conventional apparatus and methods.

The diffusion/permeation of the drug through the holes and/or pores the layers can also be further facilitated by means of using aseotropic mixtures of drugs with other harmless reagents, external pressure, or by means of inflating the below and/or above layers of the cuffs.

During the application of the apparatus, which according to a preferred embodiment of the present invention is a double cuffed ETT, the outer layer of which is perforated to let the drug leave the second layer, any desired sequences can be used for the utilization of the system. Our newly designed multilayer-cuff, as mentioned above, is installable on any ETT or other medical devices.
Figure 1: The schematic presentation of the design of the multilayer cuff of the present invention including their shape, number of layers, and positions of installation. It is noteworthy that the design of the cuff system of the present invention is not limited to the figures shown here.
   1a) Multilayer cuff according to the embodiments of the present invention where the different layers L1 - Ln, attach the devise exactly on the same point (x and y).
   1b) Multilayer cuff according to the embodiments of the present invention where the different layers L1 - Ln attach the devise exactly on different points (x 1 and y1) for L 1 and (xₙ and yₙ) for Ln.
   1c) Multilayer cuff according to the embodiments of the present invention showing a cuff installed at one end of a tube.
   1d) Multilayer cuff according to the embodiments of the present invention showing a cuff installed on a bent tube (the angle may vary).
   1e) Two multilayer cuffs according to the embodiments of the present invention installed on a linear tube (The two cuffs may be chosen to be structurally the same or different).
      In all of the figures P1-Pn show the pilot balloons of each layer (the figure only depicts the m for one of the layers), used for injecting the drugs or blowing of the air or other gases. The number of the pilot balloons may vary depending on the purposes. P'1-P'n depict the pilot balloons of each layer (the figure only depicts the m for one of the layers), used for emptying the drugs or sucking of the air or other gases. The number of the pilot balloons may vary depending on the purposes.
      PC1-PCn are pressure controlling valves attached to each pilot balloon P1-Pn and PC'1-PC'n are pressure controlling valves attached to each pilot balloon P'1-P'n.
Figure 2: An endotracheal tube (ETT) according to a preferred embodiment of the present invention having a 3-layer (L1-L3)-cuff (C), and three pilot balloons (A, D1, and D2).
Figure 3: A two cuffed endotracheal tube (ETT) according to a most preferred embodiment of the present invention having two cuffs (C₁ for Drug injection and C₂ for Fixation) and one pilot balloon (A, D) for each cuff.
Figure 4: A multilayer cuffed endotracheal tube (ETT) according to a most preferred preferred embodiment of the present invention having one double (L₁ and L₂)-cuff (C) and one pilot balloon (A, D) for each layer.

Further at least optional aspects of the invention are: The multilayer cuff is applicable for irrigation devices like Broncho Alveolar Lavage (BAL), and for PTCA (Percutaneous Transcutaneous Coronary Angioplasty), Fogarty catheters and interventional radiology devices and procedures.

The multilayer cuff can be used for injecting sclerosing agents in conditions like hemorrhoids, angiofibroma and esophageal varices, etc.

The multilayer cuff can also be used in any other medical devices in which one or several cuff(s) are used for fixation, and/or delivery of drugs to certain tissues.

The multilayer cuff is preferred to be installed or built either in the same position as the original cuff of the conventional single layer cuffed devices (Figure 1a) - d) and Figures 2 and 4) of desire or at any position before or after it (Figure 1e) and Figure 3).

The multilayer cuff is preferred to be installed or built either in the same position as the original cuff of the device of desire (Figure 1a) - d) and Figures 2 and 4) or before (proximal to) it, in the case of ETTs (Figure 1e) and Figure 3).

The cuff system of the present invention, the holes and pores of the different layers of the cuff may have any geometric distributions to meet the goals of the present invention.

The cuff can be designed to be fixed and built on the apparatus of desire, or it may be designed in the form of a multilayer, or several single layer cuffs that have the ability to be mounted on the existing designs and structures of the existing cuffed nasal or oral devices like ETTs and or devices like RAE, Bronchial blockers, double lumens, laser SLTs (Single Lumen Tubes), Supra Glottic instruments (specially during deep intubation, and/or extubation), or in order to perform irrigation in Broncho Alvolar Lavage (BAL) etc.

The cuffs can be of different shapes, sizes and can be installed or built on different middle (Figure 1a) - c) and e) or terminal (Figure 1d)) positions of the devices.

The pilot balloons and or pressure reducing valves, or in other words any part or the whole system can be used in a hand controlled or automatic manner.

The cuffs can be used in any supraglottic devices for better tolerance of patients and reducing the need for deep extubation.

The cuff can be used in special nasal airways or nasal tubes that could be designed for treatment or termination of epistaxis.

The cuff can be used in PTCA for relieving coronary stenosis (internal cuff inflation) and injection of drugs (external cuff).

The cuff can be used for irrigation such as Broncho Alveolar Lavage (BAL), etc. The single or double cuffed ETT (with intact internal cuff and perforated external cuff) can be used for elective and emergent surgeries.

The ETT using the multilayer cuff of the present invention, which can be used for prolonged infusion of drugs (Trans external cuff) for better toleration of it in ICU patients.

The path from the pilot balloon to the tip of the tube (Figures 2-4; the dotted line on the tube) can also be perforated in order to increase the delivery area.

A method of the application of the multilayer cuffs of the present invention, wherein different drugs and chemical reagents can be injected to any of the layers of the multilayer cuff(s), installed on the apparatus, in order to achieve any desired results.

Furthermore, different local anesthetics, and or other drugs of desire can be delivered to the tissues in touch with or in the vicinity of the cuff(s) with high efficiency in the form of pure drugs, pure formulations, and or as mixed with other drugs and or chemical reagents.

Different local anesthetics including lidocaine are preferred to be injected to the cuff in order to reduce the intoleration of the cuff.

Lidocaine is the most preferred local anesthetic.

The drugs can be preheated, pressurized and or mixed with other reagents to form more volatile mixtures, so that the diffusion of the drugs can be facilitated.

The features in the foregoing description, in the claims and/or in the accompanying drawings may, both and in any combination thereof, be material for realizing the invention in diverse forms thereof.

## Claims

1. A cuff (C; C₁; C₂) with a plurality of different layers (L₁, L₂, L₃; Lₘ, Lₙ), each of which being impermeable, porous or semi-permeable and/or mechanically and/or electrically and/or optically perforated.

2. Cuff according to claim 1, wherein each of the layers has at least one or several inlets and or outlets.

3. Cuff according to claim 1 or 2, wherein the number of layers is two.

4. Cuff according to any one of claims 1 to 3, wherein each layer has its own number of pilot balloons (P₁ - Pₙ, P₁' - P₂') that can be used as inlets and/or outlets of air and drugs to be injected.

5. Cuff according to any one of claims 1 to 3, wherein each layer has two pilot balloons that can be used as inlets and/or outlets of air and drugs to be injected.

6. Cuff according to any one of claims 1 to 3, wherein each layer has one pilot balloon (A; D) that is used as both the inlet and/or outlets of air and drugs to be injected.

7. Cuff according to any one of claims 1 to 6, wherein each layer has its own number of pilot balloons that can be equipped with pressure reducing valves (PC₁-PCₙ; PC₁'-PCₙ').

8. Cuff according to claim 7, wherein the pressure reducing valves are adapted to automatically reduce the pressure in the cuff to levels less than 20 mmHg over a certain threshold pressure.

9. Cuff according to any one of the preceding claims, wherein the layers are constructed of any composite, organic, inorganic, polymeric material of acceptable biocompatibility, and/or mechanical stability.

10. Cuff according to any one of the preceding claims, wherein the material to construct the lower layers or the layers not in direct contact with the body is not biocompatible.

11. Cuff according to any one of the claims 4 to 10, wherein each layer in the cuff and pilot balloon is of the same or of different materials.

12. Use of a cuff according to any one of the preceding claims singly or in various numbers on ETTs e.g. SLT (Single Lumen Tube), Double lumen, Bronchial Blocker, RAE Laser tubes, Tracheostomy tubes, supra glottic instruments and etc.

13. Method of applying a cuff according to any one of the claims 1 to 11 for delivery of a drug to target tissue, comprising the steps of: after an intubation and well before a patient is aware again, deflating an internal layer, which is intact, while a local anesthetic is injected to an external layer, following the injection of an anesthetic into the external layer, re-inflating the internal layer via its own pilot balloon which compresses the outer layer and helps in squeezing the remaining drug to reach the tracheal mucosa and anesthetizing it, removing an ETT after deflating the inner air layer preventing and eliminating emergence reactions.
